# FASCICULE DE BREVET EUROPEEN

(11) **EP 3 716 930 B1**
(45) Date de publication et mention de la délivrance du brevet: **12.01.2022**
(21) Numéro de dépôt: 18825784.4
(22) Date de dépôt: 27.11.2018
(51) Int. Cl.: A61F 13/62, A61F 13/64, A61F 13/08

(54) **DISPOSITIF CORPOREL DE COMPRESSION AVEC FERMETURE À BOUCLES ET CROCHETS**
KÖRPERKOMPRESSIONSVORRICHTUNG MIT KLETTVERSCHLUSS
BODY COMPRESSION DEVICE WITH HOOK-AND-LOOP CLOSURE

(30) Priorité: 30.11.2017 FR 1771297
(43) Date de publication de la demande: 07.10.2020
(73) Titulaire: THONIC INNOVATION, 43120 Monistrol sur Loire (FR)
(72) Inventeur: GONON, Pierre, 43120 Monistrol Sur Loire (FR)
(74) Mandataire: Gabriel, Franck
(86) Numéro de dépôt international: PCT/IB2018/059345
(87) Numéro de publication internationale: WO 2019/106531

(56) Documents cités:
- WO-A2-01/67911
- FR-A1- 2 806 904
- US-A1- 2005 132 543

## Description

### DOMAINE TECHNIQUE DE L'INVENTION

La présente invention concerne un dispositif corporel de compression destiné à être au moins partiellement enroulé sur une partie du corps et/ou un membre afin d'exercer une force de compression sur ladite partie du corps et/ou ledit membre, ledit dispositif comprenant un système de fixation avec boucles et crochets comportant une pluralité de boucles agencées sur une zone de boucles et une pluralité de crochets agencés sur une surface d'accroche.

### ETAT DE LA TECHNIQUE ANTERIEURE

Les dispositifs corporels élastiques, par exemple bandes et/ou orthèses, sont principalement utilisés pour traiter des maladies veino-lymphatiques entrainant notamment des oedèmes, des lymphoedèmes ou autres, et requérant un traitement par contention/compression. Afin de respecter au mieux les prescriptions médicales, le niveau de serrage doit être suffisant pour permettre une compression des zones à traiter. Or, en pratique, on constate que les patients appliquent les dispositifs de façon aléatoire, avec des niveaux de serrage très variables, rendant souvent les traitements peu ou pas efficaces.

Lorsque le traitement est effectué par des bas ou chaussettes de compression, l'effet de serrage obtenu résulte de la taille de la chaussette et de l'élasticité du tricot, mais ne dépend pas de la façon dont l'utilisateur enfile la chaussette. Par contre, les bandages médicaux doivent être posés par le porteur ou une personne soignante, qui enroule la bande en appliquant une certaine tension. Il est fréquent que la tension appliquée soit insuffisante et/ou irrégulière, en particulier pour des bandes de grande longueur.

Par ailleurs, un patient posant une bande sur un de ses membres peut éprouver certaines difficultés à l'appliquer correctement. En outre, lors de la pose d'une bande sur un bras, il est souvent difficile d'effectuer à la fois le serrage, l'enroulement et l'accrochage de la bande.

De nombreux exemples illustrent les efforts répétés pour parvenir à un dispositif qui soit fiable, pratique et peu coûteux. Tout d'abord, on connait les tissus étirables ayant au moins une zone de « boucles et crochets ».

Par exemple, le document FR1263391 décrit une bande de contention en tissu élastique, destinée à être enroulée autour du corps humain. La bande est réalisée de telle sorte que l'on peut faire varier le degré de contention par fixation de son extrémité libre en des emplacements déterminés et en utilisant des moyens ne risquant pas d'endommager la bande. Elle est munie d'au moins une barrette transversale en tissu spécial, notamment le tissu connu dans le commerce sous la dénomination de « Velcro », dont les poils ont leurs extrémités recourbées en forme de crochets, de façon à pouvoir s'accrocher sélectivement dans des barrettes de tissu duveteux fixées sur l'autre face de ladite bande. Ce dispositif d'accroché semble obliger l'utilisateur à respecter des zones prédéfinies pour l'accrochage, ce qui limite sa liberté d'accroché et donc de serrage.

Le document FR2806904 décrit une bande élastique destinée à bander un membre qui comprend, à son extrémité libre dans le sens de l'enroulement, une languette transversale agrippante de type tissu à boucles et crochets située sur la face de la bande tournée du côté du membre. Cette bande est munie sur sa face tournée vers l'extérieur du membre, d'au moins deux languettes transversales de réception agrippantes, de type tissu à boucles et crochets complémentaires de la languette d'extrémité, positionnées de façon telle que la languette de réception proximale la plus proche de l'extrémité de la bande est située à une distance de cette dernière inférieure ou égale au périmètre du membre le plus fin à bander et la languette de réception distale la plus éloignée de l'extrémité de la bande est située à une distance de cette dernière supérieure ou égale au périmètre du membre le plus épais à bander lorsque la bande est soumise à une tension d'utilisation. La bande peut convenir à un grand nombre de patient, cependant, son système d'accrochage en languettes semble présenter les mêmes inconvénients précités. Par ailleurs, aucun moyen ne permet de s'assurer d'un niveau convenable de serrage de la bande autour du membre, ce qui peut résulter en un bandage inefficace du fait d'un serrage trop faible.

Le document EP2250981 décrit un article polyvalent médical comprenant un bracelet en tissu élastique de forme allongée caractérisé par une extrémité distale et une extrémité proximale, une surface distale et une surface proximale, dans lequel au moins une partie de la surface proximale est vers l'extérieur munie d'une surface texturée d'un premier type, un patch absorbant fixé à la surface proximale de la sangle formant une poche entre, une pièce à main attachée à l'extrémité proximale de la sangle, au moins une partie de la surface distale vers l'extérieur munie d'une surface texturée d'un autre genre, et au moins une partie de la surface proximale vers l'extérieur munie d'une surface texturée d'un troisième type à l'extrémité proximale de celui-ci. La surface texturée de la première espèce et la surface texturée de la seconde sorte auto-adhèrent par des adjonctions physiques l'un avec l'autre formant un durcissant auto-adhérent, et la surface texturée du troisième type se caractérise par sa capacité d'auto-adhérer à la surface proximale de la sangle et/ou à la surface extérieure de la plaque absorbante y attenant. Ce système ne semble pas permettre d'avoir un niveau de serrage précis.

Les fixations de type « boucles et crochets » sont très utilisées dans l'univers médical pour fixer des bandes. Certaines fixations utilisent des crochets moulés, malgré leur complexité de fabrication et leur coût élevé.

Par exemple, Le document WO0168019 décrit des fixations extensibles réalisées à partir d'un ruban de fixation en forme de feuille qui est découpé en bandes disposées longitudinalement et séparées transversalement les unes des autres. Les bandes espacées sont fixées sur un voile élastique en forme de toile et constituent une fixation extensible. Dans certains cas, la fixation extensible est formée en continu en même temps que la bande de fixation de type feuille. Les crochets moulés sont bien séparés les uns des autres de façon à être toujours opérationnels. Cependant, la conception de ce tissu semble complexe et onéreuse. De plus, ce système ne semble pas permettre d'avoir un niveau de serrage précis.

**Certains** crochets des attaches « boucles et crochets » sont prévus avec des matériaux étirables, en général à des endroits précis.

Par exemple, le document US2002022108 décrit un système de fixation comportant des crochets moulés séparés entre eux par des zones libres. Le moulage est réalisé par des moules rigides. Pour faciliter la formation d'un produit uniforme, élastiquement extensible ou flexible, les bandes d'éléments de fixation s'étendent dans la direction de la machine de fabrication. Ce système d'accroche semble efficace, cependant, sa fabrication parait complexe et coûteuse. De plus, ce système ne semble pas permettre d'avoir un niveau de serrage précis.

Dans l'univers des couches, certains systèmes d'accroché « boucles et crochets » disposent de boucles extensibles afin de pouvoir ajuster les tailles pour un maintien efficace.

**Par** exemple, le document US2011152819 décrit un système de fixation de couche comprenant au moins une patte de connexion en élastomère disposée sur une partie latérale de la couche. La languette de connexion présente une partie liée comprenant une zone d'expansion élastomère. La zone d'expansion élastomère comprend un substrat de piqûre inélastique en matière fibreuse avec une pluralité de plis en accordéon et un réseau en zigzags de fils élastomères disposés de manière sous-jacente aux plis. Le réseau en zigzags est adapté pour appliquer des forces de contraction au substrat de couture dans la dimension de longueur de la languette de connexion. Ce système d'accroche est efficace mais ne semble pas permettre d'avoir un niveau de serrage précis. De plus, sa conception semble complexe.

Le document WO2017027830 décrit un sous-vêtement qui comprend au moins un panneau de matériau de boucle étirable. Le panneau de matériau de boucles étirable est relié à un corps principal du sous-vêtement de telle sorte qu'il peut s'étirer autour d'un côté de taille ou hanche d'un porteur. Le matériau de boucle étirable est configuré de telle sorte qu'il peut se raccorder de manière amovible à une pluralité d'éléments de prise. Les éléments de prise peuvent être des crochets d'un système de fermeture à boucles et crochets. Le sous-vêtement est configuré de telle sorte qu'il peut être facilement dissimulé sous un vêtement et peut être facilement positionné et retiré par un porteur. Un procédé pour disposer un sous-vêtement consiste à rouler ou plier le corps principal du sous-vêtement et rattacher des panneaux de matériau de boucles étirables autour de la partie roulée ou pliée pour recouvrir la partie roulée ou pliée entre les panneaux de matériau de boucles étirables. Ce système parait confortable et efficace, cependant, il ne semble pas permettre d'avoir un niveau de serrage précis.

Certains systèmes d'accroché visent à obtenir un maximum d'étirabilité et utilisent du tissu élastique.

**Par** exemple, le document WO2005004778 décrit une fermeture par anneaux et crochets comprenant un élément à crochets et un élément à anneaux. L'élément à crochets comprend un support à crochets et une pluralité de crochets s'étendant sur ledit support à crochets. Ce support à crochets contient une matière extensible. L'élément à anneaux comprend un support à anneaux et une pluralité d'anneaux s'étendant sur ledit support. Ce support à anneaux contient également une matière extensible. Ce système d'accroché parait efficace et confortable, avec un bon taux d'élasticité, cependant, sa conception semble complexe et coûteuse. De plus, ce système ne semble pas permettre d'avoir un niveau de serrage précis.

Enfin, le document US2005/0132543 décrit un tissu comprenant des zones surélevées interposées entre des zones pourvues de boucles ou crochets. Les zones en question sont dépliables, mais pas élastiques.

Tous ces différents exemples de dispositifs montrent qu'il existe un besoin pour une solution permettant de faciliter la pose de bandes de compression, tout en s'assurant que le serrage appliqué est suffisant pour obtenir les bienfaits escomptés.

Pour pallier ces différents inconvénients, l'invention prévoit différents moyens techniques.

### EXPOSE DE L'INVENTION

Tout d'abord, un premier objectif de l'invention consiste à prévoir un dispositif de compression performant et ergonomique.

Un autre objectif de l'invention consiste à prévoir un dispositif de compression simple à fabriquer et peu coûteux.

Encore un autre objectif de l'invention consiste à prévoir un dispositif de compression permettant d'améliorer le contrôle du niveau de serrage.

Encore un autre objectif de l'invention consiste à prévoir un dispositif de compression permettant d'augmenter le confort de l'utilisateur.

Enfin, un autre objectif de l'invention consiste à prévoir un dispositif de compression facile à mettre en place par un professionnel ou par un novice.

Pour ce faire, l'invention prévoit un dispositif corporel de compression destiné à être au moins partiellement enroulé sur une partie du corps et/ou un membre afin d'exercer une force de compression sur ladite partie du corps et/ou ledit membre, ledit dispositif comprenant un système de fixation avec boucles et crochets comportant une pluralité de boucles agencées sur une zone de boucles et une pluralité de crochets agencés sur une surface d'accroché, ladite surface d'accroché est élastique et susceptible d'adopter au moins deux états distincts:
- un premier état, dans lequel ladite surface d'accroché est non étirée et le tissage très fin du tissu élastique utilisé forme un champ de crochets à très forte densité et en particulier très rapprochés les uns des autres, de sorte que les crochets se croisent ensemble et sont imbriqués les uns dans les autres, empêchant l'accroche avec les boucles de la zone de boucles, et
- un second état, dans lequel ladite surface d'accroché est étirée et dans lequel les crochets sont espacés et ainsi désimbriqués les uns des autres, rendant les crochets fonctionnels et permettant l'accroche avec les boucles de la zone de boucles.

Selon une telle architecture, le dispositif, de type dit cohésif, peut être enroulé par exemple sur un membre ou une partie du corps d'un patient en fonction de la partie du corps à traiter. Dans l'état non étiré de la surface d'accroché, la coopération entre les boucles et les crochets est peu ou pas effective, tandis que dans l'état sensiblement étiré de la surface accroche, la coopération entre les boucles et les crochets est effective, rendant l'accroche fonctionnelle et efficace. En effet, dans l'état non étiré, les crochets sont imbriqués entre eux et ne sont pas ou quasiment pas opérationnels. À l'inverse, dans l'état étiré, la surface d'accroche est allongée, ce qui permet de désimbriquer les crochets les uns des autres. Cette mise à disposition des crochets est rendue possible par l'agrandissement de l'aire de la surface d'accroché. Les crochets s'écartent donc les uns des autres et le système d'accroche devient opérationnel. L'architecture permet de s'assurer que la force de serrage est suffisante pour permettre l'action thérapeutique. De plus, la force de serrage est bien répartie sur toute la longueur de la bande ou de l'orthèse.

Selon un mode de réalisation avantageux, le dispositif comprend au moins une portion en tissu comprenant une première face et une seconde face, caractérisé en ce que au moins une zone de boucles est agencée sur ladite première face du tissu, et au moins une surface d'accroche est agencée sur ladite seconde face du tissu.

Pour faciliter la mise en œuvre du dispositif, la disposition des boucles sur l'une des faces et des crochets sur l'autre face permet un enroulage et une accroche simple. La conception et l'industrialisation sont également simplifiées.

Selon un autre mode de réalisation avantageux, la surface d'accroche est mono-élastique, le sens de l'élasticité correspondant sensiblement à l'axe d'enroulement du dispositif sur ladite partie du corps.

En variante, la surface d'accroche est bi-élastique.

Si la bande n'est pas enroulée avec une force suffisante, la surface d'accroche est dans l'état non étiré, rendant la fixation impossible. Ceci permet d'éviter d'avoir un dispositif de compression posé sans tension qui serait peu ou pas efficace. A l'inverse, un serrage adéquat permet à la zone d'accroche d'être dans la position étirée, libérant ainsi les crochets et permettant la fixation du dispositif. Un niveau de serrage conforme assure que la bande ou l'orthèse est enroulée avec un niveau de serrage suffisant pour permettre la compression du membre à traiter.

De manière avantageuse, la surface d'accroche et la zone de boucles sont réalisées dans un même type de tissu.

**Ce** mode de mise en œuvre est particulièrement simple et économique et peu agressive pour la peau du patient et les autres textiles.

Egalement de manière avantageuse, le dispositif corporel comprend une partie active de compression prévue pour exercer une force de compression répartie sur le membre ou la partie du corps à traiter.

Selon un mode de réalisation avantageux, ladite partie active et le système de fixation sont réalisés avec le même tissu.

**On** constitue ainsi un dispositif corporel monopièce, simple, esthétique, et particulièrement efficace.

Selon un autre mode de réalisation avantageux, le système de fixation est venu de matière avec la partie active.

La continuité ainsi obtenue procure un produit confortable et ergonomique.

De manière avantageuse, la partie active est constituée par un matériau ou tissu différent du tissu constituant le système de fixation.

Selon un mode de réalisation avantageux, le dispositif est une bande de contention/compression.

Selon un autre mode de réalisation avantageux, le dispositif est une orthèse de contention/compression.

De manière avantageuse, le taux d'étirement potentiel de la surface d'accroche (20) est compris entre 25% et 100%, et plus préférentiellement entre 30% et 75% et encore plus préférentiellement entre 30% et 50%.

Selon encore un autre mode de réalisation avantageux, la surface d'accroche est constituée à partir d'une zone de boucles transformée en surface d'accroché, par exemple par une attaque au laser de la pluralité de boucles.

Ce type de mise en œuvre permet de réaliser un produit de conception simple, dont l'industrialisation est grandement simplifiée, en particulier lorsqu'il est mis en œuvre avec un seul et même tissu.

### DESCRIPTION DES FIGURES

**Tous** les détails de réalisation sont donnés dans la description qui suit, complétée par les figures 1a à 8b, présentées uniquement à des fins d'exemples non limitatifs, et dans lesquelles :
- les figures 1a à 1d sont des représentations schématiques illustrant un exemple de tissu comportant des zones de boucles (figure 1a) dont une zone de boucles permet de générer une surface d'accroche (figure 1b) montrée en mode non étiré (figure 1c) et en mode étiré (figure 1d) ;
- les figures 2a et 2b illustrent schématiquement les deux faces d'un exemple de dispositif de compression ;
- les figures 3, 4a à 4e et 5 sont des représentations schématiques montrant des variantes de réalisation de dispositif de compression de type bande de contention;
- les figures 6a à 8 b sont des représentations schématiques montrant divers exemples de réalisation du dispositif de compression de type orthèse de contention.

### DESCRIPTION DETAILLEE DE L'INVENTION

### EXEMPLE DE MISE EN ŒUVRE D'UN SYSTEME DE FIXATION AVEC BOUCLES ET CROCHETS

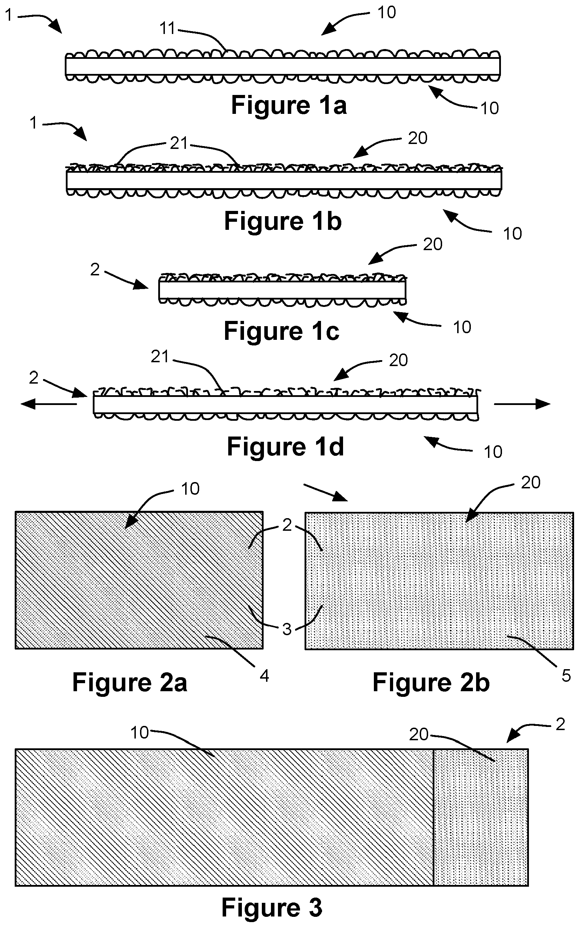

Les figures 1a à 1d illustrent de façon schématique à partir d'un exemple d'un tissu élastique 1 comportant initialement des zones de boucles 10 sur chacune de ses faces, un exemple de mise en œuvre d'un système de fixation 3 à boucles et crochets utilisés dans des dispositifs de compression décrits en relation avec les figures 2a à 8b.

Dans sont état initial, le tissu 1 comprend de chaque côté une pluralité de boucles 11 formées dans des zones de boucles 10 comme présenté à la figure 1a.

La figure 1b illustre schématiquement la transformation effectuée à la zone de boucles 10 de la face supérieure pour obtenir une zone d'accroche 20. Les fils formant les boucles 11 sont traités, par exemple à l'aide d'une attaque au laser, pour les transformer au moins partiellement en crochets 21. Le traitement mis en œuvre sur le tissu permet qu'au moins une portion des boucles du tissu élastique soient modifiées. Par exemple, les boucles sont coupées en au moins deux parties, les parties restantes de la boucle demeurant fixées à la trame du tissu, et forment des crochets 21. Le traitement permet l'obtention des extrémités inclinées ou repliées des crochets.

Le tissage très fin du tissu élastique utilisé permet l'obtention d'un « champ » de crochets à très forte densité et en particulier très rapprochés les uns des autres, de sorte que les crochets se croisent ensemble. Dans une telle configuration, correspondant au mode non étiré, les crochets sont peu ou pas efficaces pour attraper d'éventuelles boucles. La figure 1c illustre un exemple d'agencement des crochets 21 répartis sur une surface d'accroche 20 à l'état non étiré.

Le tissu 1 est étirable au moins dans le sens longitudinal. Cette caractéristique permet d'espacer les crochets 21 et donc de les désimbriquer les uns des autres lorsque la surface d'accroche 20 se trouve dans son état étiré, comme présenté à la figure 1d. Dans cet état, les crochets sont fonctionnels ou efficaces, et sont aptes à saisir ou attraper d'éventuelles boucles. Le niveau d'étirement de la surface d'accroche pour favoriser un bon fonctionnement des crochets est avantageusement supérieur à 30% et plus préférentiellement compris entre 30% et 50%.

Le système de fixation 3 est ainsi remarquable en ce qu'il comporte deux états distincts, intrinsèquement liés à l'élasticité du tissu constituant le système.

**Ce** système procure par ailleurs un niveau de fixation particulièrement élevé car, une fois les boucles accrochées dans les crochets (en mode étiré), le tissu est ensuite relâché pour retrouver un état sensiblement non étiré. Dans cet état, les crochets sont de nouveaux rapprochés les uns des autres et créent ainsi une sorte de verrouillage des crochets, rendant la séparation des boucles et crochets plus difficile, et réalisable grâce à un effort de séparation « relativement élevé » (?). Pour détacher les boucles et crochets, la surface d'accroche est de préférence étirée. Cette précaution permet de faciliter la séparation des boucles et crochets et protège les boucles et les crochets contre des détériorations prématurées.

Par ailleurs, la forte densité de crochets et leur agencement de façon aléatoire permet d'optimiser au maximum l'accrochage du système de fixation 3.

### PREMIER EXEMPLE DE PRODUIT UTILISANT UN SYSTEME DE FIXATION AVEC BOUCLES ET CROCHETS : BANDE DE CONTENTION/COMPRESSION

Les figures 2a à 5 illustrent de façon schématique des exemples de bandes de contention 2 comprenant au moins des portions réalisées avec un tissu 1 similaire à celui utilisé aux figures 1a à 1d pour réaliser le système de fixation 3 préalablement décrit.

Le dispositif corporel 2 comprend au moins une portion en tissu 1 comprenant deux faces 4 et 5. Une zone de boucles 10 est agencée sur la première face 4, représentée schématiquement à la figure 2a. Une surface d'accroche 20 est agencée sur la seconde face 5, représentée schématiquement à la figure 2b.

De cette façon, l'enroulage de la bande de contention est particulièrement aisé car peu importe l'endroit où arrive l'extrémité libre de la surface d'accroche 20 sur la zone de boucles 10, l'accroche est possible. En effet, avec ce mode de réalisation, toute partie de la seconde face 5 peut être fixée à n'importe quelle partie de la première face 4, à condition que la surface d'accroche 20 soit étirée, pour permettre la mise à disposition des crochets 21 qui peuvent se fixer sur les boucles 11 de la zone de boucle 10.

Par ailleurs, cette caractéristique permet d'avoir un système de fixation 3 en continu sur tout le long de la bande de contention, par exemple, lorsque l'utilisateur superpose en partie la bande lors de l'enroulage.

Afin de traiter de façon efficace des symptômes tels qu'une mauvaise circulation veineuse et/ou lymphatique par exemple, la bande de contention doit être appliquée avec un niveau de serrage suffisant, et si possible appliqué de façon uniforme sur toute la longueur de la bande. Le système de fixation 3, grâce à ses deux états, permet d'éviter un serrage insuffisant car la surface d'accroche 20 doit être étirée pour que les crochets 21 puissent saisir les boucles 11 et donc maintenir le dispositif en place.

La figure 3 montre schématiquement un autre exemple de bande de contention 2, Dans cet exemple, le système de fixation 3 est configuré avec une surface d'accroche 20 agencée au niveau de l'extrémité libre de la bande. Le reste de la bande est constitué de zones de boucles 10, tel qu'illustré à la figure 3. Cette configuration permet à l'utilisateur de fixer la bande sur n'importe quelle zone de boucles 10. Le dispositif est donc adapté à toutes les morphologies et pratiquement toutes les parties du corps à traiter.

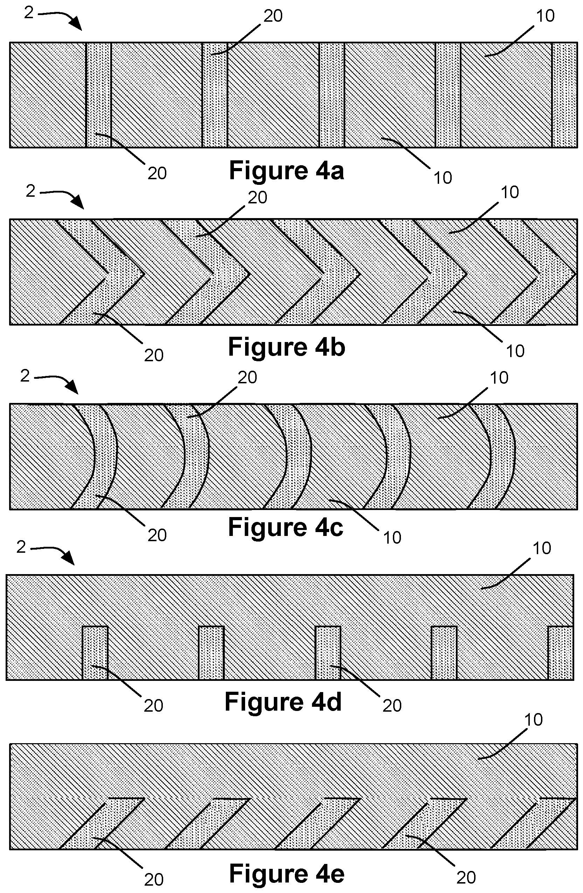

Dans l'exemple de réalisation de la figure 4a, des zones de boucles 10 et des surfaces d'accroche 20 sont agencées en alternance le long de la bande. Cette configuration permet de multiplier les secteurs d'accrochage afin d'avoir un meilleur maintien sans pour autant avoir une surface faite uniquement de crochets 21.

Les figures 4b et 4c présentent des variantes de réalisation dans lesquelles des zones de boucles 10 sont agencées en forme de chevron ou d'arc de cercle.

Les figures 4d et 4e présentent des variantes de réalisation dans lesquelles des zones de boucles 10 sont agencées sur une fraction de la largeur de la bande. Dans les exemples illustrés, la fraction correspond à la moitié de la largeur. En variante, des fractions différentes sont prévues, couvrant par exemple entre 1/5 et 4/5 de la largeur de la bande. Ces modes de réalisations sont avantageux dans les cas où la bande est enroulée autour d'un membre du patient, l'enroulement étant mis en œuvre avec un recouvrement partiel d'un tour à l'autre.

Afin de réduire les coûts et de simplifier la mise en œuvre, la zone de boucles 10 et la surface d'accroche 20 peuvent être réalisées dans la même matière et/ou le même type de tissu.

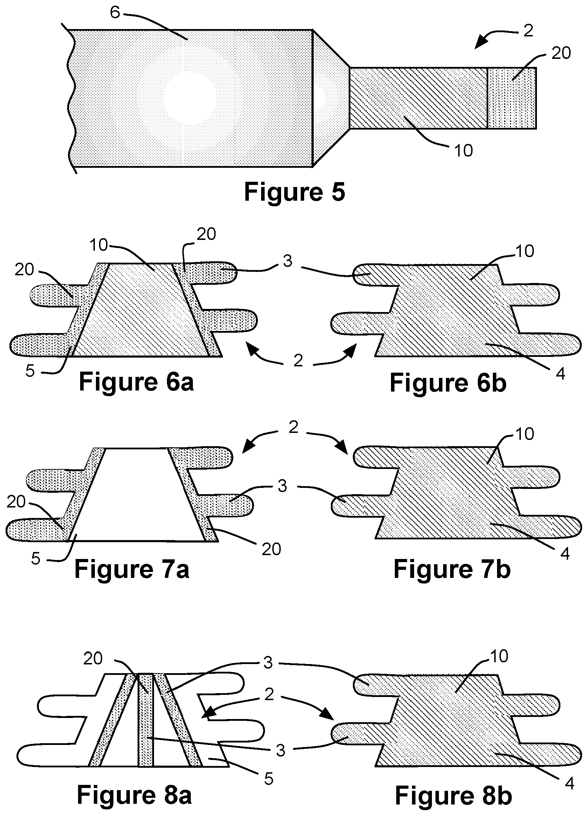

En variante, tel qu'illustré à la figure 5, la bande de contention 2 comprend une partie active 6 prévue pour exercer un effort de compression une fois la bande convenablement installée. La partie active peut en variante être réalisée dans le même matériau que le système de fixation 3. Cette caractéristique permet d'avoir une fabrication mono-pièce, afin de simplifier la fabrication et de diminuer le coût tout en ayant un dispositif efficace. Le système de fixation 3 de la bande de contention peut également être venu de matière avec la partie active 6 de compression, ce qui permet d'obtenir un produit simple de conception, confortable et efficace.

De plus, cette caractéristique permet d'avoir une pièce unique, sans raccord entre les différentes zones de boucles 10 et les surfaces d'accroche 20, qui, dans une utilisation extrême par exemple, pourraient fragiliser le système de fixation 3.

La force de contention des bandes est mesurée à l'aide d'un dynamomètre. Par exemple, après trois cycles de fatigue de 0 % à 30 %, on enregistre la force nécessaire pour allonger à 30 % la bande élastique lors du dernier cycle (selon la méthode ITFH). En fonction de leur force de contention, les bandes peuvent être partagées en différents niveaux, comme par exemple de 20 à 45 cN/cm, de 46 à 100 cN/cm, de 101 à 160 cN/cm, et supérieur à 160 cN/cm.

### SECOND EXEMPLE DE PRODUIT UTILISANT UN SYSTEME DE FIXATION AVEC BOUCLES ET CROCHETS : ORTHESE DE CONTENTION/COMPRESSION

Les figures 6a à 8 b illustrent de façon schématique des exemples de réalisation dans lesquels le dispositif corporel 2 de compression est une orthèse de contention. L'orthèse comprend un corps central cylindrique et des pattes latérales, prévues pour faciliter la fixation à un membre du corps par enroulement autour de ce dernier.

Dans tous les exemples, la figure de droite illustre une première face 4 et la figure de gauche présente la seconde face 5. Dans tous ces exemples, la première face 4 est recouverte d'une zone de boucles 10.

Dans le premier exemple (figure 6a), la seconde face 5 comprend une zone de boucles 10 aménagée au niveau du corps central et des surfaces d'accrochés 20, de chaque côté ainsi que sur les pattes latérales.

Dans le second exemple (figure 7a), la seconde face 5 comprend une zone libre aménagée au niveau du corps central et des surfaces d'accrochés 20, de chaque côté ainsi que sur les pattes latérales.

Enfin, le dernier exemple (figure 8a) prévoit une pluralité de zones d'accroche 20 réparties sur le corps central et des surfaces libres, de chaque côté ainsi que sur les pattes latérales. De multiples variantes de réalisation de l'orthèse sont possibles, par exemple avec des pattes latérales sur un seul côté, ou sans patte latérale.

Le fonctionnement et la mise en œuvre des exemples d'orthèse illustrés sont similaires aux bandes de contention préalablement décrites, à l'exception du fait que les orthèses forment un enroulement sur un seul tour. Tel que préalablement décrit pour les bandes de contention, le système de fixation 3 à deux états permet de s'assurer que les orthèses sont appliquées avec un niveau de serrage suffisant. En cas de serrage insuffisant ou en absence de serrage, le système de fixation demeure inopérant et l'orthèse ne peut pas être fixée.

### Numéros de référence employés sur les figures

- 1: Tissu
- 2: Dispositif corporel de compression
- 3: Système de fixation
- 4: Première face
- 5: Seconde face
- 6: Partie active de compression

- 10: Zone de boucles
- 11: Boucles

- 20: Surface d'accroche
- 21: Crochets

## Revendications

1. Dispositif corporel (2) de compression destiné à être au moins partiellement enroulé sur une partie du corps et/ou un membre afin d'exercer une force de compression sur ladite partie du corps et/ou ledit membre, ledit dispositif comprenant un système de fixation (3) avec boucles et crochets comportant une pluralité de boucles (11) agencées sur une zone de boucles (10) et une pluralité de crochets (21) agencés sur une surface d'accroche (20), **caractérisé en ce que** ladite surface d'accroche (20) est élastique et susceptible d'adopter au moins deux états distincts:
- un premier état, dans lequel ladite surface d'accroche (20) est non étirée et le tissage très fin du tissu élastique utilisé forme un champ de crochets à très forte densité et en particulier très rapprochés les uns des autres, de sorte que les crochets se croisent ensemble et sont imbriqués les uns dans les autres, empêchant l'accroche avec les boucles (11) de la zone de boucles (10), et
- un second état, dans lequel ladite surface d'accroche (20) est étirée et dans lequel les crochets (21) sont espacés et ainsi désimbriqués les uns des autres, rendant les crochets fonctionnels et permettant l'accroche avec les boucles (11) de la zone de boucles (10).

2. Dispositif corporel (2) de compression selon la revendication 1, dans lequel le dispositif comprend au moins une portion en tissu (1) comprenant une première face (4) et une seconde face (5), **caractérisé en ce que** au moins une zone de boucles (10) est agencée sur ladite première face (4) du tissu, et au moins une surface d'accroche (20) est agencée sur ladite seconde face (5) du tissu.

3. Dispositif corporel (2) de compression selon l'une des revendications 1 ou 2, dans lequel ladite surface d'accroche (20) est mono-élastique, le sens de l'élasticité correspondant sensiblement à l'axe d'enroulement du dispositif sur ladite partie du corps.

4. Dispositif corporel (2) de compression selon l'une quelconque des revendications précédentes, dans lequel la surface d'accroche (20) et la zone de boucles (10) sont réalisées dans un même type de tissu.

5. Dispositif corporel (2) de compression selon l'une quelconque des revendications précédentes, comprenant une partie active (6) de compression prévue pour exercer une force de compression répartie sur le membre ou la partie du corps à traiter.

6. Dispositif corporel (2) de compression selon la revendication 5, dans lequel ladite partie active (6) de compression et le système de fixation (3) sont réalisés avec le même tissu.

7. Dispositif corporel (2) de compression selon l'une des revendications 5 ou 6, dans lequel le système de fixation (3) est venu de matière avec la partie active (6).

8. Dispositif corporel (2) de compression selon la revendication 5, dans lequel la partie active (6) est constituée par un matériau ou tissu différent du tissu (1) constituant le système de fixation (3).

9. Dispositif corporel (2) de compression selon l'une quelconque des revendications 1 à 8, dans lequel le dispositif est une bande de contention/compression.

10. Dispositif corporel (2) de compression selon l'une quelconque des revendications 1 à 8, dans lequel le dispositif est une orthèse de contention/compression.

11. Dispositif corporel (2) de compression selon l'une quelconque des revendications précédentes, dans lequel le taux d'étirement potentiel de la surface d'accroche (20) est compris entre 25% et 100%, et plus préférentiellement entre 30% et 75% et encore plus préférentiellement entre 30% et 50%.

12. Dispositif corporel (2) de compression selon l'une quelconque des revendications précédentes, dans lequel la surface d'accroche (20) est constituée à partir d'une zone de boucles (10) transformée en surface d'accroche (20).

## Patentansprüche

1. Körperkompressionsvorrichtung (2), die dazu bestimmt ist, zumindest teilweise über einen Körperteil und/oder ein Körperglied gewickelt zu werden, um eine Kompressionskraft auf den Körperteil und/oder das Körperglied auszuüben, wobei die Vorrichtung ein Befestigungssystem (3) mit Schlaufen und Haken umfasst, das mehrere in einem Schlaufenbereich (10) angeordnete Schlaufen (11) und mehrere auf einer Hakenfläche (20) angeordnete Haken (21) aufweist, **dadurch gekennzeichnet, dass** die Hakenfläche (20) elastisch ist und zumindest zwei verschiedene Zustände einnehmen kann:
- einen ersten Zustand, in dem die Hakenfläche (20) ungedehnt ist und das sehr feine Gewebe des verwendeten elastischen Stoffs ein Feld von Haken ausbildet, die mit sehr hoher Dichte und insbesondere sehr eng beieinanderliegend angeordnet sind, so dass die Haken sich verkreuzen und ineinander verschachtelt sind, wodurch ein Verhaken mit den Schlaufen (11) des Schlaufenbereichs (10) verhindert wird, und
- einen zweiten Zustand, in dem die Hakenfläche (20) gedehnt ist und in dem die Haken (21) voneinander beabstandet und somit entschachtelt sind, wodurch sie funktionell werden und ein Verhaken mit den Schlaufen (11) des Schlaufenbereichs (10) ermöglichen.

2. Körperkompressionsvorrichtung (2) nach Anspruch 1, wobei die Vorrichtung zumindest einen Stoffabschnitt (1) mit einer ersten Seite (4) und einer zweiten Seite (5) umfasst, **dadurch gekennzeichnet, dass** auf der ersten Seite (4) des Stoffs zumindest ein Schlaufenbereich (10) angeordnet ist und auf der zweiten Seite (5) des Stoffs zumindest eine Hakenfläche (20) angeordnet ist.

3. Körperkompressionsvorrichtung (2) nach einem der Ansprüche 1 oder 2, wobei die Hakenfläche (20) monoelastisch ist, wobei die Elastizitätsrichtung im Wesentlichen der Wickelachse der Vorrichtung auf dem Körperteil entspricht.

4. Körperkompressionsvorrichtung (2) nach einem der vorangehenden Ansprüche, wobei die Hakenfläche (20) und der Schlaufenbereich (10) aus derselben Stoffart gefertigt sind.

5. Körperkompressionsvorrichtung (2) nach einem der vorangehenden Ansprüche, umfassend einen aktiven Kompressionsteil (6), der dazu vorgesehen ist, eine verteilte Kompressionskraft auf das zu behandelnde Körperglied bzw. den zu behandelnden Körperteil auszuüben.

6. Körperkompressionsvorrichtung (2) nach Anspruch 5, wobei der aktive Kompressionsteil (6) und das Befestigungssystem (3) aus demselben Stoff gefertigt sind.

7. Körperkompressionsvorrichtung (2) nach einem der Ansprüche 5 oder 6, wobei das Befestigungssystem (3) mit dem aktiven Teil (6) einstückig ausgebildet ist.

8. Körperkompressionsvorrichtung (2) nach Anspruch 5, wobei der aktive Teil (6) aus einem anderen Material oder Stoff ausgebildet ist als der Stoff (1), der das Befestigungssystem (3) bildet.

9. Körperkompressionsvorrichtung (2) nach einem der Ansprüche 1 bis 8, wobei die Vorrichtung ein Stütz-/Kompressionsverband ist.

10. Körperkompressionsvorrichtung (2) nach einem der Ansprüche 1 bis 8, wobei die Vorrichtung eine Stütz-/Kompressionsorthese ist.

11. Körperkompressionsvorrichtung (2) nach einem der vorangehenden Ansprüche, wobei der potentielle Dehnungsgrad der Hakenfläche (20) zwischen 25 % und 100 %, und vorzugsweise zwischen 30 % und 75 % und besonders vorzugsweise zwischen 30 % und 50 % liegt.

12. Körperkompressionsvorrichtung (2) nach einem der vorangehenden Ansprüche, wobei die Hakenfläche (20) aus einem zu einer Hakenfläche (20) umgewandelten Schlaufenbereich (10) ausgebildet ist.

## Claims

1. A body compression device (2) intended to be at least partially wound over a part of the body and/or a limb in order to exert a compression force on said part of the body and/or said limb, said device comprising a fixing system (3) with loops and hooks comprising a plurality of loops (11) arranged on a loop zone (10) and a plurality of hooks (21) arranged on a hooking surface (20), **characterized in that** said hooking surface (20) is elastic and can adopt at least two distinct states:
- a first state, in which said hooking surface (20) is unstretched and the very fine weave of the elastic fabric used forms a field of hooks with very high density and in particular that are very close to one another, such that the hooks cross one another and are nested in one another, preventing hooking with the loops (11) of the loop zone (10), and
- a second state, in which said hooking surface (20) is stretched and in which the hooks (21) are spaced apart and thus no longer nested in one another, rendering the hooks functional and allowing hooking with the loops (11) of the loop zone (10).

2. The body compression device (2) as claimed in claim 1, wherein the device comprises at least one portion made of fabric (1) comprising a first face (4) and a second face (5), **characterized in that** at least one loop zone (10) is arranged on said first face (4) of the fabric, and at least one hooking surface (20) is arranged on said second face (5) of the fabric.

3. The body compression device (2) as claimed in one of claims 1 or 2, wherein said hooking surface (20) is mono-elastic, the direction of elasticity corresponding substantially to the axis of winding of the device on said part of the body.

4. The body compression device (2) as claimed in any one of the preceding claims, wherein the hooking surface (20) and the loop zone (10) are produced in a same type of fabric.

5. The body compression device (2) as claimed in any one of the preceding claims, comprising an active compression part (6) provided to exert a compression force distributed over the limb or the part of the body to be treated.

6. The body compression device (2) as claimed in claim 5, wherein said active compression part (6) and the fixing system (3) are produced with the same fabric.

7. The body compression device (2) as claimed in one of claims 5 or 6, wherein the fixing system (3) is made of a piece with the active part (6).

8. The body compression device (2) as claimed in claim 5, wherein the active part (6) is composed of a material or fabric different from the fabric (1) of which the fixing system (3) is composed.

9. The body compression device (2) as claimed in any one of claims 1 to 8, wherein the device is a splinting/compression band.

10. The body compression device (2) as claimed in any one of claims 1 to 8, wherein the device is a splinting/compression orthosis.

11. The body compression device (2) as claimed in any one of preceding claims, wherein the potential stretch rate of the hooking surface (20) lies between 25% and 100%, and more preferentially between 30% and 75% and even more preferentially between 30% and 50%.

12. The body compression device (2) as claimed in any one of preceding claims, wherein the hooking surface (20) is composed of a loop zone (10) transformed into a hooking surface (20).
